# EUROPEAN PATENT APPLICATION

(11) **EP 1 745 761 A1**
(43) Date of publication of application: **24.01.2007**
(21) Application number: 06021208.1
(22) Date of filing: 23.08.2001
(51) Int. Cl.: A61F 2/06, A61B 5/103, A61B 5/107

(54) **Method of manufacturing custom intravascular devices**

(30) Priority: 23.08.2000 US 644640
(62) Divisional of application: 01966090.1
(71) Applicant: LeMaitre Acquisition LLC, Burlington MA 01803 (US)
(72) Inventor: Colone, William M., Phoenix AZ 85044 (US); Diethrich, Edward B., Paradise Valley AZ 85253 (US); Teeter, Barbara L., Phoenix AZ 85044 (US); Farl, Kevin G., Phoenix AZ 85044 (US); Creer, William L., Phoenix AZ 85048 (US)
(74) Representative: Carter, Stephen John

(57) **Abstract**

A method for manufacturing a custom-sized intravascular device includes the steps of (1) measuring the diameter of a diseased vessel, and (2) manufacturing the custom-sized intravascular device according to the diameter. Optionally, the method includes the steps of measuring a plurality of diameters of the diseased vessel and a length along the vessel, and manufacturing the custom-sized intravascular device according to the plurality of diameters and length. Also enclosed herein is a method of performing a surgical procedure utilizing a custom-sized intravascular device manufactured according to the invention.

## Description

### FIELD OF THE INVENTION

The present invention relates to intravascular devices that are custom-sized to fit a particular diseased vessel or vessels within a patient.

### BACKGROUND OF THE INVENTION

The present invention relates to a method and apparatus for the manufacture of an intravascular device, such as an endoluminal stent or stent graft, suited for delivery into the vascular system of a patient. As is known by those skilled in the art, the term "lumen" refers to a channel or cavity within an organ, such as an artery or other blood vessel. Each of the terms "intravascular device" "endoluminal device," "intraluminal device" and "endoprosthesis" means a device designed for placement inside of a blood vessel, i.e., in the lumen of the blood vessel, and such device can be a stent, stent graft, occluder, pump, catheter or other device. The term "transluminal," as used herein, refers to a device that connects the lumen of one vessel to the lumen of another. The term "occlude" means closed off or blocked, and an "occluder" is a device designed to close off or block a lumen. As used herein, the term "vessel" or "blood vessel" refers to a structure, such as an artery or vein, that transports blood. A "catheter" is a generally tubular, surgical instrument inserted into a body cavity, such as a lumen.

A stent is a device that provides support to a weakened or diseased vessel. Stents are usually circular wires that apply outward pressure against a vessel to keep the vessel open. Stents may be used to repair compromised coronary arteries which have become narrowed (called a stenosis) or altogether blocked by the build up of plaque. Stents may also be used to support structures that are being anastomosed.

A stent covered or lined with biocompatible material is known as a stent graft, prosthetic vascular graft or endoluminal graft. Known stent grafts are tubular structures (either right-cylindrical tubes or other shapes, such as conical) that allow for the passage of fluid, such as blood, through the stent graft. For example, prosthetic vascular grafts formed of biocompatible materials (e.g., Dacron or expanded, porous polytetrafluoroethylene (PTFE) tubing) have been employed to replace or bypass occluded or damaged blood vessels. They may also be used to repair or replace weakened or diseased blood vessels, such as the aorta, which have developed dilated (or enlarged), weakened areas known as aneurysms. In the aorta, aneurysms may often occur in the areas where the aorta divides into two secondary arteries, such as the two common iliac arteries, which supply blood to the lower limbs. Stent grafts are disposed in aneurysms to remove the pressure on the weakened blood vessel to reduce the risk of rupture, which is when the natural wall bursts. The use of stents and stent grafts for treatment or isolation of vascular aneurysms and vessel walls which have been thinned or thickened by disease (this use is called endoluminal repair or exclusion) is known. Examples of stent grafts are described in U.S. Patent Nos. 4,955,899 and 5,152,782.

There are many types of stents and stent grafts. For example, stents and stent grafts that are "self-expanding," i.e., that are inserted into the vascular system in a compressed or contracted state, and permitted to expand upon removal of a restraint, are known. Some self-expanding stents and stent grafts employ a wire of suitable material, such as stainless steel or nitinol (nickel-titanium), configured to provide an outward radial force. Such a stent typically has a tubular configuration and a slightly greater diameter than the diameter of the lumen in which it is intended to be used.

Some stents are generally flexible so they can be easily maneuvered through the various body vessels for deployment. Once in position, the stent may be deployed by allowing the stent to expand to its uncompressed state or by expanding the stent by use of a catheter balloon. Various forms of stents and/or stent grafts are described in U.S. Patent Nos. 5,873,906, 5,302,317, 5,662,713, 5,575,816, 5,0507,767, 5,415,664, 4,800,882, 4,907,336, and 5,718,724.

The use of trigger or release wires to control expansion of a self-expanding endoprosthesis are known. Additionally, U.S. Patent No. 5,415,664 describes a stent delivery and deployment apparatus including three concentric tubes, an interior hollow tube and an outer sheath, and an inner tubular actuation member with a cup-like gripping member rigidly attached to its distal end. Other devices for deploying self-expanding endoprosthesis are described in U.S. Patent Nos. 5,484,444, 5,833, 5,776,142, 5,873,906, and 5,700,269.

A vascular stent which comprises a length of sinuous or "zigzag" wire formed into a helix is disclosed in U.S. Patent No. 4,886,062. The helix defines a generally cylindrical wall which, in use, constitutes a prosthetic intraluminal wall. The sinuous configuration of the wire permits radial expansion and compression of the stent. The patent discloses that the stent can be delivered percutaneously and expanded in situ using a balloon catheter.

An expandable intraluminal stent graft which is constituted by a tubular member formed from a plurality of intersecting elongate members which permit radial expansion and compression of the stent is disclosed in U.S. Patent No. 4,733,665.

An intraluminal stent which is constituted by a sinuous wire formed into a helix is disclosed in EP-A-0556850. Juxtaposed apices of the wire are secured to one another so that each hoop of the helix is supported by its neighboring hoops to increase the overall strength of the stent and to minimize the risk of plaque herniation. In some embodiments the stent of EP-A-0556850 further comprises a tubular graft member to form an endoluminal prosthesis.

Intravascular devices, such as stents, stent grafts and occluders, are deployed using different methods. They may be deployed using a "cut-down" procedure, i.e., cutting directly into the lumen from an entry point proximate to the site where the prosthesis is to be deployed and placing the device into the lumen. Alternatively, they may be deployed using a less invasive percutaneous method, such as cutting through the skin to access a lumen at a convenient, and relatively low-trauma, entry point, and routing the device through the lumen until the site where the device is to be deployed is reached. Deployment of an intravascular device is sometimes effected using a delivery catheter with coaxial inner (plunger) and outer (sheath) tubes arranged for relative axial movement. The device is compressed and disposed within the distal end of the outer catheter tube in front of the inner tube. The distal end of the catheter is then maneuvered, typically routed though a lumen, until it (and thus the intravascular device) is positioned in the vicinity of the intended treatment site. The inner tube is then held stationary when the outer tube of the delivery catheter is withdrawn. The inner tube prevents the intravascular device from being withdrawn with the outer tube, so that, as the outer tube is withdrawn, the intravascular device radially expands into a substantially conforming surface contact with the interior of the lumen. An example of such a delivery system is described in U.S. Patent No. 4,655,771, the disclosure of which is incorporated herein by reference.

It is also known to insert and advance an intravascular device in the form of a unitary bifurcated graft through a single branch of the femoral arterial system, to a point beyond the treatment site, then pull or draw one of the limbs into the contralateral (opposite) branch by manipulation of a contralateral-femoral wire catheter or snare. Such a system is described in U. S. Patent 5,639,278, the disclosure of which is incorporated herein by reference. An example of another deployment system is one that requires cross-femoral wire catheter and guide wires, and is described in U.S. Patent No. 5,489,295 and PCT Application No. WO 98/36708. It is also suggested in U.S. Patent No. 4,617,932 that blood flow entering the graft can be utilized to cause the graft to float free in the blood stream so that it may be directed to the proper position.

The application of bifurcated stent grafts to branched lumen (such as the infrarenal portion of the aortic artery where it bifurcates to the common iliac arteries) is also known. However, the deployment of a bifurcated stent graft is typically relatively invasive because the respective portions of bifurcated stent grafts often must be joined in situ and require a plurality of catheterizations. Stent grafts for bifurcated lumen are described in U.S. Patent Nos. 5,906,640, 5,755,734, and 5,827,320. These devices require large access ports in a portion of the vessel that is usually much deeper under the skin than the preferred femoral artery entry site.

The respective disclosures of the following U.S. Patents and patent applications are incorporated herein by reference: U.S. Patent Application No. 09/401,599 entitled "Delivery System for Self-expanding Stents and Grafts," filed on September 22, 1999; U.S. Patent Application No. 09/361,192 entitled "A Balloon-assisted Intraluminal Stent Graft," filed on July 26, 1999; U.S. Patent Application No. 09/574,870 entitled "Expandable Vascular Prosthesis" filed on May 19, 2000; and U.S. Patent Application No. 09/244,343 entitled "A Method of Making Large Diameter Vascular Prosthesis Made by Said Method," filed on February 4, 1999.

The prior art intravascular devices are generally satisfactory for the treatment of aneurysms, stenoses and other angeological diseases at sites in blood vessels, as long as an intravascular device is available to properly fit the dimensions of the blood vessel requiring repair. The prior art does not disclose, however, intravascular devices that are custom-sized to fit the lumen of a particular vessel.

An intravascular device must accurately fit the vessel lumen requiring repair or the repair may not be adequate. Further, the more closely the dimensions of the intravascular device fit the lumen of the diseased vessel, the less surgical intervention required to achieve a proper fit. Because of the desirability to have an intravascular device that closely fits the dimensions of a vessel requiring repair, intravascular device manufacturers often manufacture numerous sizes (i.e., different widths and lengths) of intravascular devices to fit different sized diseased vessels.. Further, medical facilities often stock numerous sizes. Despite the many sizes manufactured and stocked some diseased or weakened vessels develop a shape for which there is no available intravascular device. Currently, such vessels can only be repaired using highly invasive surgical techniques that require at least several weeks of convalescence.

### SUMMARY OF THE INVENTION

The present invention solves the above-referenced problems. An intravascular device according to the method of the invention is custom-sized to fit the diseased vessel. Consequently, diseased vessels that heretofore could not be adequately treated using standard-sized intravascular devices, and that could only be treated utilizing invasive surgical procedures, can be treated utilizing the invention. Further, the invention simplifies or eliminates inventory management and the need for specialized knowledge of intravascular device sizes by hospital personnel or doctors. Because each device is custom sized for each vessel no inventory must be maintained and no selection is required by medical personnel.

The benefits are (1) less time spent by medical personnel on inventory management and device selection, and (2) an intravascular device properly sized to fit the vessel requiring repair. Further, unusual sized lumens, for which there are currently no available intravascular devices, can be repaired utilizing the present invention without the need for highly invasive surgery.

Disclosed herein are (1) a method for manufacturing a custom-sized intravascular device, (2) custom-sized intravascular devices made by the method, and (3) a method of performing a surgical procedure using a custom-sized intravascular device of the invention.

### BRIEF DESCRIPTION OF THE DRAWINGS

The preferred exemplary embodiment of the present invention will hereinafter be described in conjunction with the appended drawing, where like designations denote like elements, and:
Figure 1 depicts a diseased blood vessel.
Figure 1A shows side views of custom-sized intravascular devices manufactured according to the invention for the repair of the vessel shown in Fig. 1.
Figure 2 depicts another diseased blood vessel.
Figure 2A shows a side view of a custom-sized intravascular device manufactured according to the invention for the repair of the vessel shown in Fig. 2.
Figure 3 depicts another diseased blood vessel including a custom-sized intravascular device according to the invention.
Figure 4 depicts another diseased blood vessel including a custom-sized intravascular device according to the invention.
Figure 5 depicts another diseased blood.
Figure 5A shows a side view of custom-sized intravascular devices according to the invention for the repair of the vessel shown in Fig. 5.
Figure 6 depicts measurements taken from another diseased blood vessel.
Figure 6A is a side view of a custom-sized intravascular device manufactured to repair the vessel shown in Fig. 6.
Figure 6B is a side view of a mandrel used to manufacture the custom-sized intravascular device shown in Figure 6A.

### DETAILED DESCRIPTION OF A PREFERRED EMBODIMENT

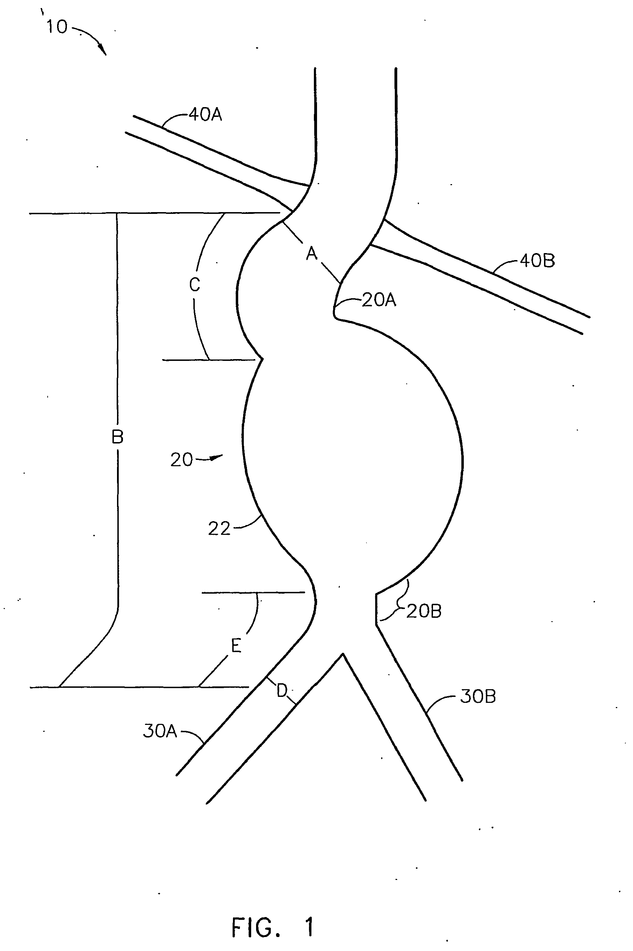

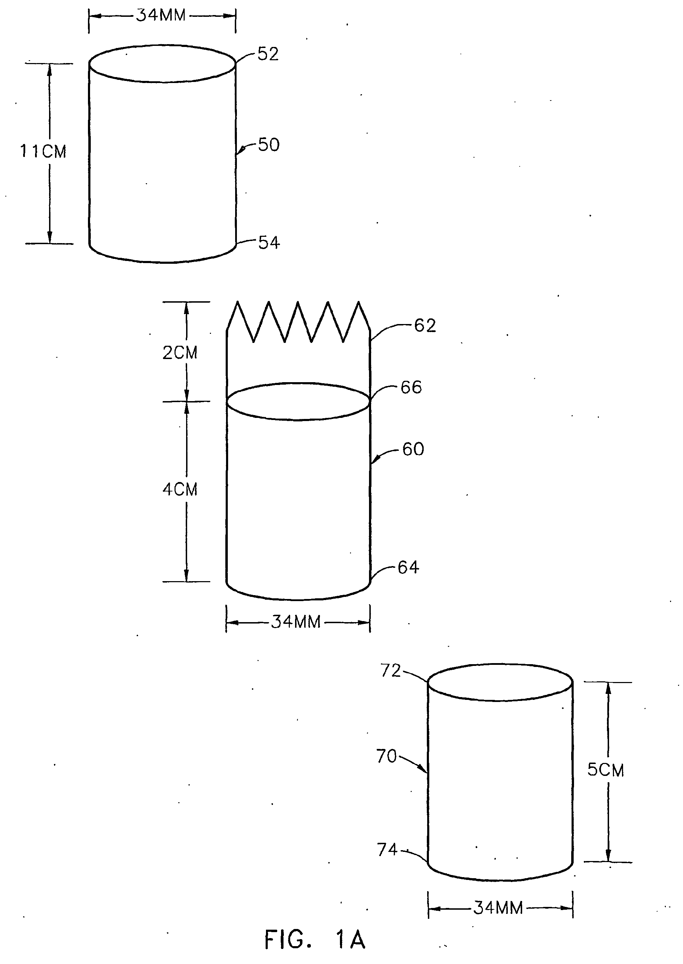

Turning now to the drawings where the purpose is to describe a preferred embodiment of the invention and not to limit same, Fig. 1 shows a diseased or weakened blood vessel that can receive a custom-sized intravascular device made according to the method of the invention. As used herein, the term "vessel," when used to define the invention, shall mean any vessel for which a custom-sized intravascular device according to the invention is to be implanted, for any reason, such as disease or because the vessel has become weak.

Generally, the method according to the invention includes the following steps: (1) measuring one or more dimensions of a vessel lumen, or one or more dimensions of each of a plurality of vessel lumen, and (2) producing an intraluminal device, or plurality of intraluminal devices, according to the dimensions of the vessel(s) lumen(s). Measuring the lumen(s) can be accomplished by any suitable method. For example, the dimension(s) may be measured by an angiographical technique (x-ray examinations of blood vessels or lymphatics following the injection of a radiopaque substance). This is usually done by injecting radiopaque dye into a vessel and photographing the dye with an X-ray machine as it moves through the vessel. It is also known to use Computerized Tomography (CT) scans and the like to measure arterial diameters from which the length of intravascular devices can be extrapolated. Other more novel methods for visualizing a vessel, and thus measuring its diameter and length, include spiral CT scan and intravascular ultrasound (IVUS). In many cases, only three dimensions need be known to produce a custom-sized device according to the invention: (1) the diameter of the lumen at which one end (the first end) of the intravascular device will be placed (for example, this may be the diameter of the lumen of the distal neck of an aorta); (2) the diameter of the lumen at which the second end of the intravascular device will be placed (for example, this may be the diameter of the lumen of one of the iliac arteries or the diameter of the lumen of the proximal neck of the aorta), and (3) the length between the locations at which the diameters referenced in (1) and (2) above are measured. Other dimensions, however, may be measured, and in some cases other dimensions may have to be measured.

The measurements are normally made by a physician, typically the physician that will deploy the intravascular devices made according to the inventor. The decision about which measurements to take, at what location(s) in the vessel(s) to take them, and any instructions provided the manufacturer of the custom-sized intravascular device with respect to the final dimensions of the intravascular device, is usually left to the physician.

The measurements are then preferably transmitted from a medical (first) facility to a manufacturing (second) facility using any suitable method. Facsimile transmission, e-mail, mail, overnight delivery, courier, telephone or any other method can be used. It is possible, however, that the manufacture could take place at the same facility in which the dimensions were measured and/or that the device could be deployed at the facility that manufactures the custom-sized intravascular device.

Once the dimensions of the lumen(s) are known, one or more custom-sized intravascular devices according to the invention can be produced using any suitable technique, such techniques being known to those skilled in the art. If the device is a stent or stent graft, mandrels are preferably produced using techniques known to those skilled in the art. The stent graft is then cast on the mandrel. In other instances, the stent graft may be hand crafted using tubing, stents and any means to affix the two. Furthermore, an intravascular device according to the invention may be bifurcated. For example, it may include a single passage that extends through the aorta and branch into two passages to transfer blood into each of the iliac arteries.

As is known to those skilled in the art, the dimensions of an intraluminal device are usually slightly larger than the dimensions of the vessel in order to create a pressure fit (or, in the case of a stent, the intraluminal device is larger than the lumen because it is designed to push outward against the lumen wall and enlarge the lumen). Therefore, an intraluminal device according to the invention is generally not manufactured to the exact diameter of the lumen, but is preferably slightly oversized to create a pressure fit when deployed.

As used herein, the term "deployed" refers to any method for placing a custom-sized intravascular device according to the invention into the lumen of a vessel requiring repair. A custom-sized intraluminal device according to the invention may be deployed using any suitable technique. For example, it could be deployed using the previously mentioned cut-down procedure, any type of delivery catheter, or any other method.

The preferred embodiments of the invention shall be explained by reference to the following examples.

### EXAMPLE 1

Fig. 1 shows a blood vessel network 10 that has a weakened or diseased aorta 20. Aorta 20 has a distal neck 20A, a proximal neck 20B and a dilated section 22 wherein the wall of aorta 20 has expanded to form an aneurysm. The common iliac arteries 30A and 30B branch off beneath aorta 20 and the renal arteries 40A and 40B branch to either side above aorta 20. To manufacture a custom-sized intravascular device for aorta 20 each of the following was measured: (1) the diameter A of distal neck 20A of aorta 20 beneath renal arteries 40A, 40B, (2) a length B which includes the length of aneurysm 22 plus an added distance beneath and above aneurysm 22 because the intravascular device is preferably pressure fit against nondiseased lumen of a vessel (either the proximal neck 20B of aorta 20 or iliac artery 30A), (3) the length C of distal neck 20A from aneurysm 22 to renal arteries 40A, 40B, (4) the diameter D of iliac artery 30A, (5) length E, which includes the length of the proximal neck 20B of aorta 20, and (6) part of the length of iliac artery 30A. As used herein, the term "diameter" refers to the width of a blood vessel lumen. The blood vessel lumen may not be cylindrical, so the width may not be uniform. Therefore, the term diameter, as used herein refers to both a single width of a lumen, which is currently what is measured, and the cross-sectional dimensions of a noncylindrical lumen. After the measurements were taken they were transmitted to a second facility by facsimile where custom-sized intravascular devices were manufactured according to the dimensions to fit and repair vessel 20.

Fig. 1A shows three custom-sized intravascular devices manufactured to fit the vessel shown in Fig. 1. Device 50 is a stent graft having an outer diameter of 34 mm and a length of 11 cm. Device 60 is a stent graft open at the top. Device 60 includes a graft (or covered) portion 64, a stent (or uncovered) portion 62 and markers 66. Device 60 has a total length of 6 cm and a width of 34 mm. Device 70 is an optional stent graft having a length of 5 cm and a width of 34 mm.

Each stent (or open end of a stent graft) referred to herein is preferably comprised of nitinol wire having a diameter of .018 inches. Each stent graft referred to herein is preferably comprised of nitinol wire having a diameter of .018 inches surrounded by PTFE. The preferred stent and stent graft are described in more detail in U.S. Patent Application No. 09/244,343 to William M. Colone et al., entitled "A Method of Making Large Diameter Vascular Prostheses and a Vascular Prosthesis made by Said Method" and filed on February 4, 1999, the disclosure of which is incorporated herein by reference.

Devices 50, 60 and (optionally) 70 are placed inside of aorta 20 and iliac artery 30A such that they overlap to create a single conduit for the transfer of blood. Generally, the intraluminal devices are deployed from top to bottom, with the uppermost device being deployed first and the lower most device being deployed last. In this case, device 60 is first positioned in distal neck 20A to create an anchor for device 50. The open end 62 of device 60 can be in the path of renal arteries 40A, 40B without blocking the blood flow to arteries 40A, 40B. Preferably device 60 includes a marker 66 that is visible by the physician when utilizing an imaging process to position the device. The purpose of marker 66 is to avoid positioning closed portion 64 in the path of renal arteries 40A, 40B, which could seriously obstruct the blood flow to arteries 40A, 40B.

Device 50 is positioned next Preferably, distal end 52 of device 50 overlaps (it fits inside of) portion 64 of device 60 to create a continuous passage for the transfer of blood. Device 50 extends through dilated portion 22, into proximal section 20B, and preferably into iliac artery 30A. If, during the course of the deployment, it is discovered that extra length is needed to property anchor proximal end 54 of device 50, device 70 may be deployed. Device 70 is optional and is sometimes referred to as a cuff or an extension. If used, end 72 of device 70 overlaps with (it fits inside of) proximal end 54 of device 50 to create a continuous passage for the transfer of blood, and end 74 forms a pressure fit against the lumen of artery 30A to anchor it and device 50 in place. Not shown here is a transluminal graft that would be used to transfer part of the blood from artery 30A to artery 30B and an occulder that would be positioned in artery 30B between the transluminal graft and proximal end 20B of aorta 20 in order to eliminate any back pressure. (The width of artery 30B and preferably the length from (1) the position of the transluminal graft in artery 30B, to (2) proximal and 20B of aorta 20, should be measured to manufacture a custom-sized occluder according to the invention.)

### EXAMPLE 2

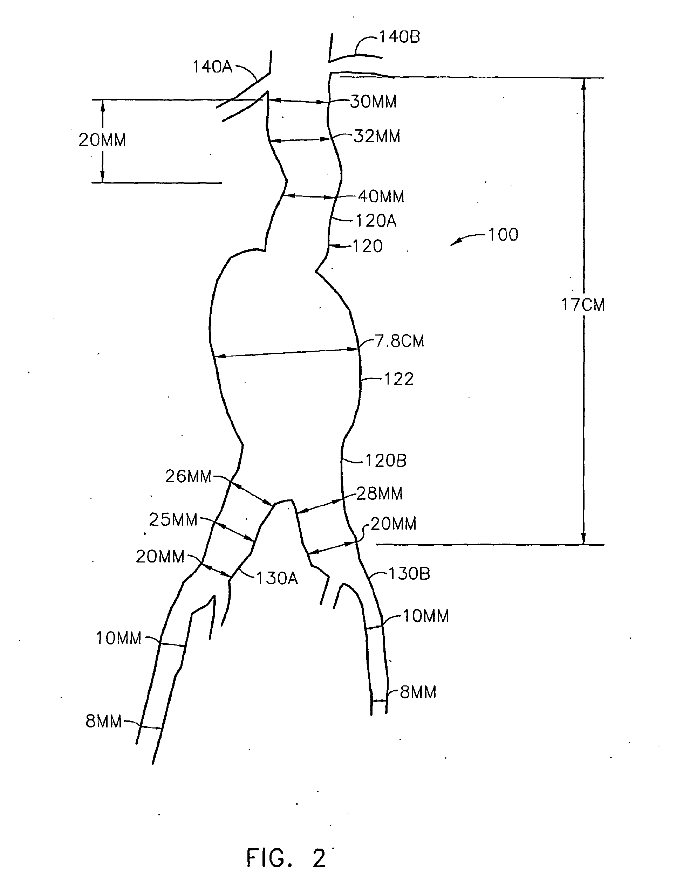

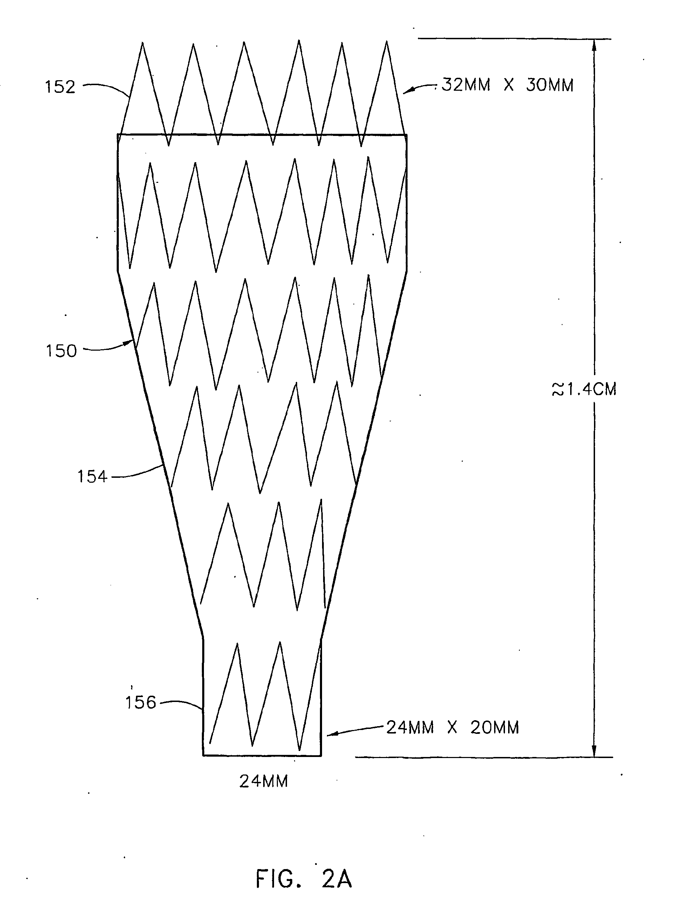

Fig. 2 shows another diseased or weakened blood vessel network 100. Vessel network 100 includes an aorta 120, having a distal neck 120A, a proximal neck 120B, and a dilated portion 122 that has formed an aneurysm. Vessel network 100 also includes iliac arteries 130A, 130B and renal arteries 140A, 140B. As can be seen in Fig. 2, numerous widths and lengths were measured for aorta 120 and iliac arteries 130A, 130B. These measurements were transmitted by facsimile to a second facility for the manufacture of custom-sized intravascular devices to fit and repair vessel 120.

Fig. 2A is a custom-sized, cone-shaped stent graft 150 made in accordance with the invention to fit vessel 120. Stent graft 150 has a first end 152 that is open (i.e., end 152 is an uncovered, wire stent), so as to not block the flow of blood into renal arteries 140A, 140B. Main body 154 is closed and has a second end 156.

In use, open end 152 is positioned in distal neck 120A and may be positioned in the path of renal arteries 140A, 140B. Body portion 154 extends through dilated portion 122 and end 156 is positioned in, and is pressure fit against the lumen of, iliac artery 130A. Not shown here is a transluminal graft that would be used to transfer part of the blood from artery 130A to artery 130B and an occluder that would be positioned in artery 130B between the transluminal graft and proximal end 120B of aorta 120 in order to eliminate any back pressure. (The width of artery 130B and preferably the length from (1) the position of the transluminal graft in artery 30B, to (2) proximal end 20B of aorta 20, should be measured to manufacture a custom-sized occluder according to the invention.)

### EXAMPLE 3

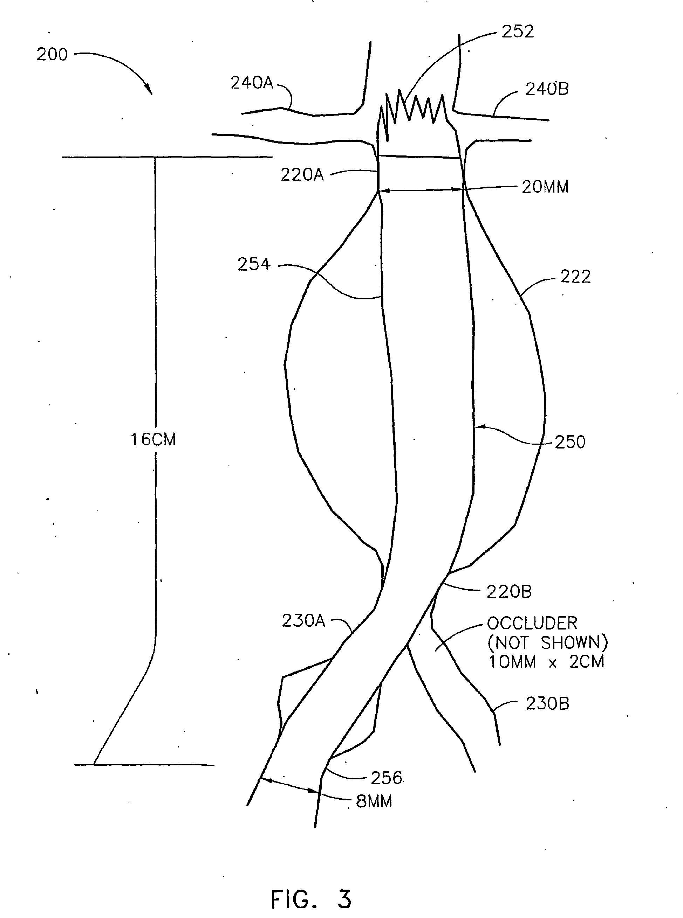

Fig. 3 shows a custom-sized stent graft 250 according to the invention deployed in vessel network 200. Network 200 includes an aorta 220 having a distal neck 220A, a proximal neck 220B and a dilated portion 222 that forms an aneurysm. Network 200 also includes iliac arteries 230A, 230B and renal arteries 240A, 240B. Measurements were taken of the: (1) width of the distal neck 220A, (2) the length from distal neck 220A to a position in iliac artery 230A, (3) the width of iliac artery 230A, and the width of iliac artery 230B. The attending physician decides, based on his experience and the facilities available at his disposal, the preferred form and location of each measurement. Based on his/her judgment, a custom-sized stent or stent graft will be specified. In this case, once measured, the dimensions were transmitted to a second facility by facsimile where custom-sized intravascular devices according to the invention were produced.

A 10 mm diameter by 2 cm in length custom-sized occluder (not shown) was manufactured and deployed in iliac artery 230B to eliminate back pressure from iliac artery 230B into aorta 220.

Intravascular device 250 was also manufactured and deployed as shown in aorta 220 and iliac 230A. Device 250 has an open end 252, a closed main body 254 and a closed end 256. Device 250 extends from aorta 220 immediately below renal arteries 240A, 240B and into iliac artery 230A. Open end 252 as shown, extends into the path of renal arteries 240A, 240B. Closed end 256 is positioned in the lumen of iliac 230A. Not shown here is a transluminal graft that would be used to transfer part of the blood from artery 230A to artery 230B.

### EXAMPLE 4

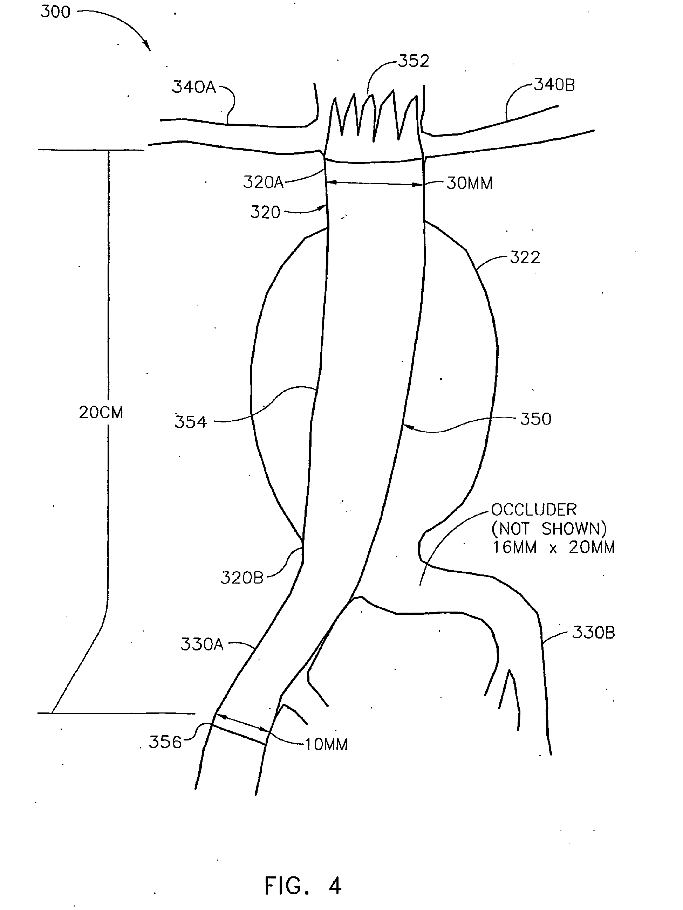

Fig. 4 depicts a custom-sized intravascular device according to the invention deployed in a blood vessel network 300. Network 300 includes an aorta 320 having a distal neck 320A, a proximal neck 320B and a dilated portion 322 that forms an aneurysm. Network 300 also includes iliac arteries 330A, 330B and renal arteries 340A, 340B. Measurements were taken of the: (1) width of the distal neck 320A, (2) the length from distal neck 320A to a position in iliac artery 230A, (3) the width of iliac artery 330A, and the width of iliac artery 330B. These dimensions were transmitted to a second facility via facsimile where custom-sized intravascular devices according to the invention were produced.

A 16 mm diameter by 2 cm in length custom-sized occluder (not shown) was manufactured and deployed in iliac artery 330B to eliminate back pressure from iliac artery 330B into aorta 320.

Intravascular device 350 was also manufactured and deployed as depicted in aorta 320 and iliac 330A. Device 350 has an open end 352, a closed main body 354 and a closed end 356. Device 350 extends from aorta 320 immediately below renal arteries 340A, 340B and into iliac artery 330A. Open end 352 as shown, extends into the path of renal arteries 340A, 340B. Closed end 356 is positioned in the lumen of iliac 330A. Not shown here is a transluminal graft that would be used to transfer part of the blood from artery 330A to artery 330B.

### EXAMPLE 5

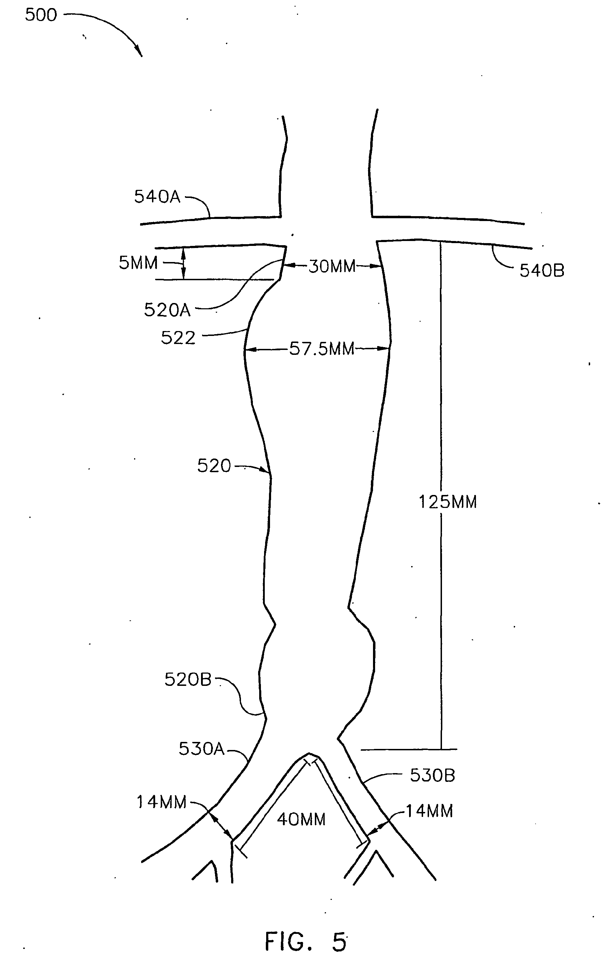

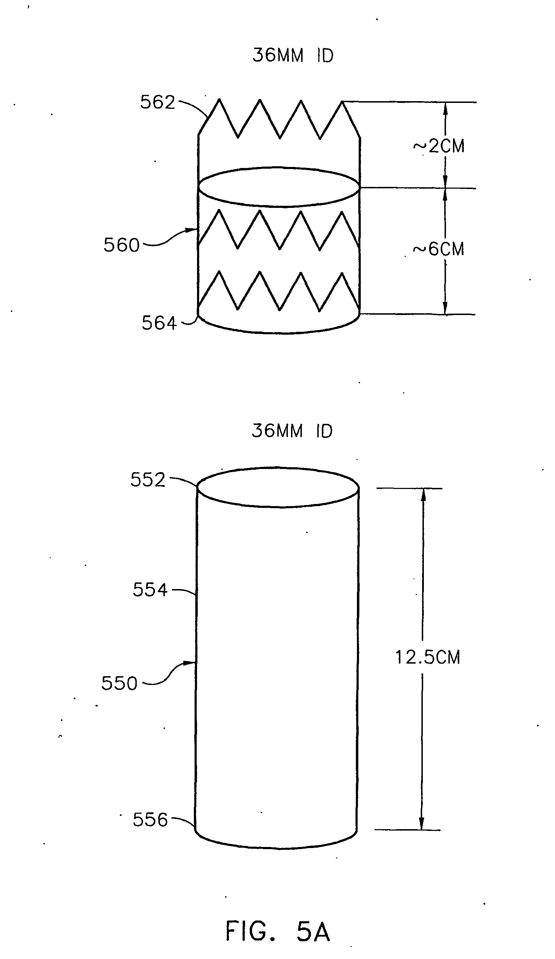

Fig. 5 shows a diseased blood vessel network 500 that includes aorta 520. Aorta 520 includes distal neck 520A, proximal neck 520B and a dilated portion 522 that has formed an aneurysm. Vessel network 500 further includes renal arteries 540A, 540B and iliac arteries 530A, 530B. As shown in Fig. 5, several measurements were taken at various locations on vessel network 500. Fig. 5A shows custom-sized intravascular device 550 and 560 made to repair aorta 520. Device 550 is a stent graft made to extend from distal neck 520A to at least proximal neck 520B, and preferably into iliac artery 530A. Open stent graft 560 has a first portion 562 and a second portion 564. First portion 562 is open (i.e., it is a wire stent) and can be positioned in the path of renal arteries 530A, 530B without blocking the flow thereto. Portion 564 is a stent graft and is positioned beneath renal arteries 530A, 530B to create a pressure fit against the lumen of aorta 520 at distal end 520A. Device 550 has a first end 552, a body 554 and a second end 556. End 552 is overlapped (i.e., placed inside of) portion 564 in order to create a continuous passage for the transfer of blood. Graft 550 extends through dilated section 522 and into at least proximal end 520B of aorta 520. Preferably, second end 556 of graft 550 extends into iliac artery 530A where it forms a pressure fit against the lumen of artery 530A. If it does not extend into artery 530A, the physician has the option of adding a properly sized extension or cuff of the type previously described.

### EXAMPLE 6

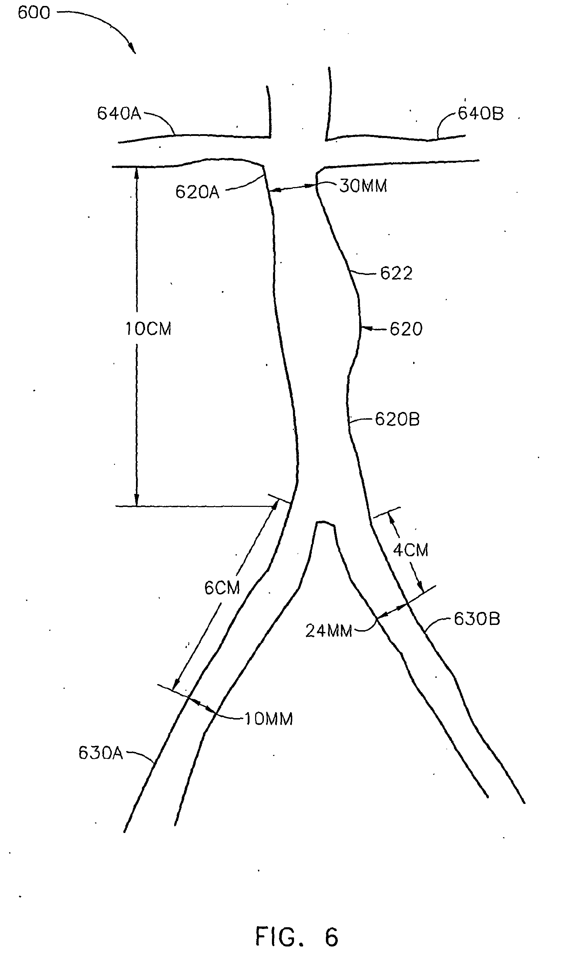

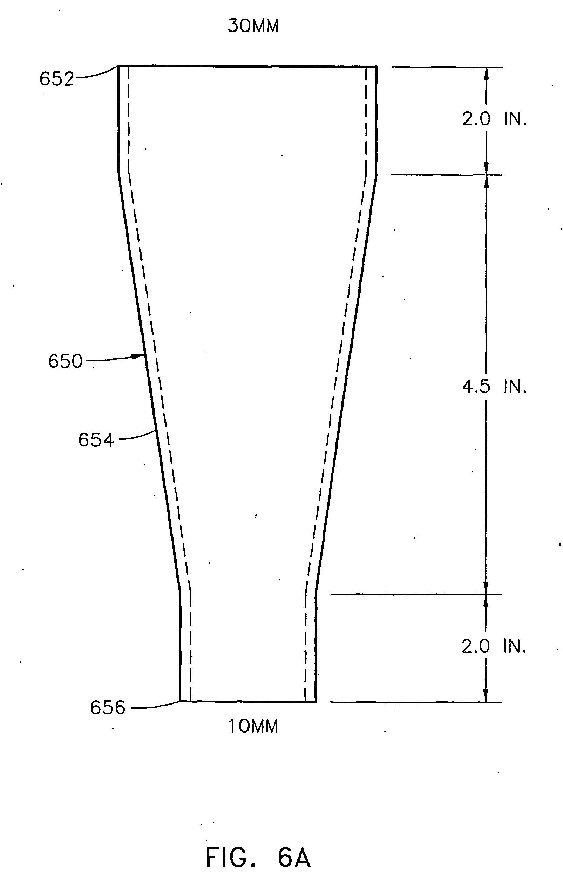

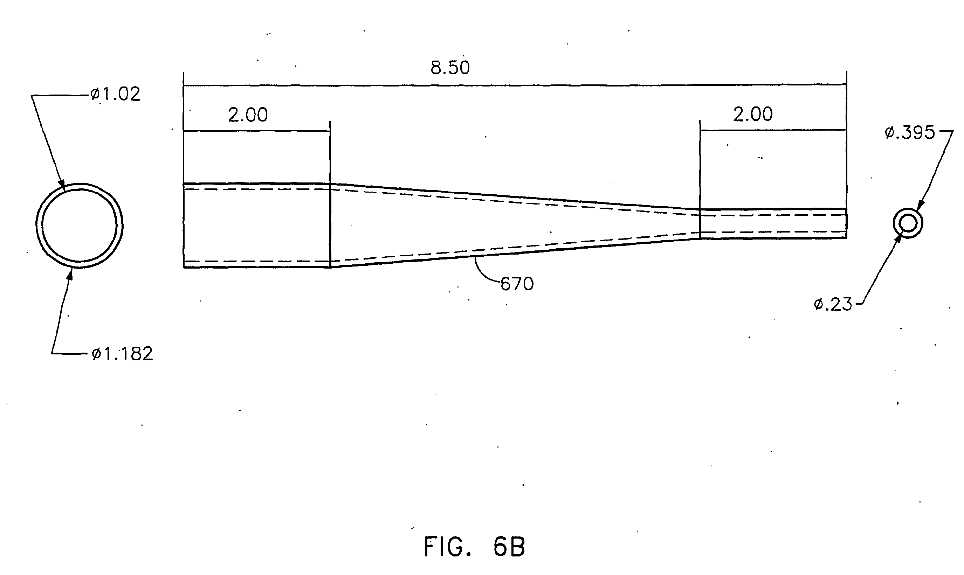

Fig. 6 illustrates measurements taken of a vessel network (not shown). The measurements included the (1) diameter of the distal neck of the aorta, (2) length from the renal arteries to the iliac artery, (3) length into the first iliac artery, where the graft would terminate and form a pressure fit against the lumen of the iliac artery, (4) the width of the second iliac artery, and (5) a length along the second iliac artery. Fig. 6A shows a custom-sized stent graft 650 manufactured in accordance with these dimensions. Stent graft 650 includes a closed first end 652, a closed body 654 and a closed second end 656. Graft 650 is designed to extend from the distal neck of the aorta and into the first iliac artery of the vessel network.

Fig. 6B shows a mandrel 670 that was custom manufactured in order to produce the stent graft shown in Fig. 6A. The preferred method for manufacturing the mandrel is disclosed in U.S. Patent Application No. 09/244,343 to William M. Colone, entitled "A Method of Making Large Diameter Vascular Prostheses and a Vascular Prostheses Made by Said Method" and filed on February 4, 1999, the disclosure of which is incorporated herein by reference. Any suitable method could be used, however, to manufacture mandrel 670 or to produce device 650.

Having now described preferred embodiments of the invention, alterations and modifications that do not depart from the spirit of the invention will occur to others. The invention is thus not limited to the preferred embodiments, but is set forth in the following claims and legal equivalents thereof. Unless specifically stated otherwise in the claims or the specification, (1) any method or device according to the claimed invention may include additional steps or structures, and (2) method steps may be performed in any order suitable for producing a device according to the invention.

The following numbered paragraphs set out various aspects and features of the disclosure of this application, which aspects and features may form the basis of claims in this application and/or future divisional applications.
1. A method of manufacturing a custom-sized intravascular device, the method including the steps of measuring a diameter of a vessel and manufacturing a custom-sized intravascular device according to the diameter.
2. The method of paragraph 1 which further includes the steps of measuring more than one diameter of a vessel and manufacturing the custom-sized intravascular device according to the more than one diameter.
3. The method of paragraph 1 which further includes the step of measuring the length of the diseased vessel and manufacturing a custom-sized intravascular device according to the diameter and length.
4. The method of paragraph 1 which further includes the steps of:
   (a) measuring more than one diameter of a vessel and measuring a length along the vessel; and
   (b) manufacturing the custom-sized intravascular device according to the more than one diameter and the length.
5. The method of paragraph 1 wherein the diameter is measured by an angiogram.
6. The method of paragraph 1 wherein the diameter is measured by an MRI scan.
7. The method of paragraph 1 wherein the diameter is measured by a CT scan.
8. The method of paragraph 1 wherein the diameter is measured by intravascular imaging.
9. The method of paragraph 1 wherein the diameter is measured by an ultrasound scan.
10. The method of paragraph 1 wherein the intravascular device is a stent.
11. The method of paragraph 1 wherein the intravascular device is an occluder.
12. The method of paragraph 1 wherein the intravascular device is a stent graft.
13. The method of paragraph 10 wherein the stent is self expanding.
14. The method of paragraph 10 wherein the stent is balloon expandable.
15. The method of paragraph 12 wherein the stent graft is self expanding.
16. The method of paragraph 12 wherein the stent graft is balloon expandable.
17. The method of paragraph 1 which further includes the steps of producing a custom-sized mandrel according to the diameter, the mandrel being produced prior to the manufacture of the custom-sized intravascular device.
18. The method of paragraph 12 wherein the intravascular device includes a plastic graft material.
19. The method of paragraph 18 wherein the graft material is polytetrafluoroethylene.
20. The method of paragraph 18 wherein the graft material is Dacron.
21. The method of paragraph 12 wherein the intravascular device has two ends and further includes an open stent at one end.
22. The method of paragraph 21 wherein the open stent is comprised of wire.
23. The method of paragraph 1 wherein the vessel includes a coronary artery.
24. The method of paragraph 1 wherein the vessel includes the aorta.
25. The method of paragraph 1 wherein the vessel includes the superficial femoral artery.
26. The method of paragraph 1 wherein the vessel includes a saphenous vein bypass graft.
27. The method of paragraph 24 wherein the vessel further includes one or more of the iliac arteries.
28. The method of paragraph 10 wherein stent is comprised of metal.
29. The method of paragraph 28 wherein stent is comprised of nitinol.
30. The method of paragraph 18 wherein the stent graft further comprises nitinol wire.
31. The method of paragraph 1 which further includes the steps of measuring the diameter at one facility and transmitting the diameter to a second facility where the custom-sized intravascular device is manufactured.
32. The method of paragraph 31 wherein the width is transmitted from one facility to the second facility via facsimile transmission.
33. The method of paragraph 31 wherein the diameter is transmitted from one facility to the second facility via the Internet.
34. The method of paragraph 31 wherein the diameter is transmitted from one facility to the second facility via E-mail.
35. The method of paragraph 31 wherein the diameter is transmitted from one facility to the second facility via telephone.
36. The method of paragraph 31 wherein the diameter is transmitted from one facility to the second facility via express mail service.
37. A method of producing one or more custom-sized intravascular devices for use in a plurality of vessels, the method comprising the steps of:
   (a) measuring a diameter of each of the plurality of vessels; and
   (b) manufacturing the one or more custom-sized intravascular devices according to the diameters.
38. The method of paragraph 37 wherein one custom-sized intravascular device is manufactured.
39. The method of paragraph 38 in which the custom-sized intravascular device is bifurcated.
40. The method of paragraph 37 wherein a plurality of custom-sized intravascular devices are manufactured.
41. The method of paragraph 37 which further includes the step of manufacturing one or more mandrels used for manufacturing the one or more custom-sized intravascular devices.
42. The method of paragraph 40 wherein one of the custom-sized intravascular devices is a stent graft and another of the custom-sized intravascular devices is an occluder.
43. The method of paragraph 37 wherein the plurality of vessels is the aorta and one of the iliac arteries.
44. The method of paragraph 37 wherein the plurality of vessels is the aorta and both of the iliac arteries.
45. The method of paragraph 37 wherein one of the plurality of vessels is the coronary artery.
46. The method of paragraph 37 which further includes the steps of measuring a length along one or more of the plurality of vessels and manufacturing the one or more custom-sized intravascular devices according to the diameters and the length.
47. The method of paragraph 37 which further includes the steps of:
   (a) measuring a diameter of each of the plurality of vessels and measuring, for one of the plurality of vessels, a second diameter and a lengths; and
   (b) manufacturing the one or more custom-sized intravascular devices according to the diameters and length.
48. The method of paragraph 37 wherein the one or more intravascular devices includes a stent.
49. The method of paragraph 37 wherein one or more the intravascular devices includes occluder.
50. The method of paragraph 37 wherein one or more intravascular devices includes a stent graft.
51. The method of paragraph 48 wherein the stent is self expanding.
52. The method of paragraph 48 wherein the stent is balloon expandable.
53. The method of paragraph 50 wherein the intravascular device includes plastic graft material.
54. The method of paragraph 53 wherein the graft material is polytetrafluoroethylene.
55. The method of paragraph 53 wherein the graft material is Dacron.
56. The method of paragraph 50 wherein the stent graft has two ends and includes an open stent at one end.
57. The method of paragraph 53 wherein the stent graft further comprises nitinol wire.
58. The method of paragraph 42 wherein another of the custom-sized intravascular devices is a cuff.
59. The method of paragraph 42 wherein the stent graft has a first end and a second end and includes an open stent at one end.
60. A method of performing a medical procedure on a patient, the method comprising the steps of:
   (a) measuring a diameter of a vessel;
   (b) manufacturing a custom-sized intravascular device according to the diameter; and
   (c) deploying the custom intravascular device into the vessel.
61. The method of paragraph 60 which further includes the step of anesthetizing the patient prior to inserting the custom-sized intravascular device.
62. The method of paragraph 60 wherein the patient is a human.
63. The method of paragraph 60 wherein the diameter of the vessel is measured in a first procedure and the custom-sized intravascular device is inserted in a second procedure.
64. The method of paragraph 60 which further includes the steps of:
   (a) measuring more than one diameter of a vessel and measuring a length along the vessel; and
   (b) manufacturing the custom-sized intravascular device according to the more than one diameter and the length.
65. The method of paragraph 60 wherein the diameter is measured by an angiogram.
66. The method of paragraph 60 wherein the diameter is measured by an MRI scan.
67. The method of paragraph 60 wherein the diameter is measured by a CT scan.
68. The method of paragraph 60 wherein the diameter is measured by an ultrasound scan.
69. The method of paragraph 60 wherein the intravascular device is a stent graft.
70. The method of paragraph 69 wherein the stent graft has a plastic graft material.
71. The method of paragraph 70 wherein the graft material is polytetrafluoroethylene.
72. The method of paragraph 70 wherein the graft material is Dacron.
73. The method of paragraph 70 wherein the stent graft includes nitinol wire.
74. The method of paragraph 60 wherein the intravascular device is a stent.
75. The method of paragraph 60 wherein the intravascular device is an occluder.
76. The method of paragraph 74 wherein the intravascular device is self-expanding.
77. The method of paragraph 74 wherein the intravascular device is balloon expandable.

## Claims

1. A method of manufacturing a custom sized intravascular device, the method comprising:
producing the custom sized intravascular device according to dimensions of a vessel lumen, the dimensions transmitted from a first facility to a second facility and the dimensions including (1) a first diameter of the vessel lumen at which a first end of the custom sized intravascular device is to be placed, (2) a second diameter of the vessel lumen at which a second end of the custom sized intravascular device is to be placed, and (3) the length between the first end and the second end.

2. The method of claim 1 wherein the custom sized intravascular device is custom sized to fit a diseased vessel.

3. The method of any of the preceding claims wherein the custom sized intravascular device is manufactured oversize to create a pressure fit when deployed.

4. The method of any of the preceding claims wherein the custom sized intravascular device is a stent graft.

5. The method of claim 4 wherein the custom sized intravascular device includes an uncovered, wire stent portion.

6. The method of any of the preceding claims wherein the custom sized intravascular device is a cone-shaped stent graft.

7. The method of any of claims 1 to 5 wherein the custom sized intravascular device is a cylindrical shaped stent graft.

8. The method of claim 1 wherein the first diameter is the diameter of a distal neck of an aorta.

9. The method of claim 1 wherein the second diameter is the diameter of an iliac artery.

10. The method of claim 1 wherein the second diameter is the diameter of a proximal neck of an aorta.

11. The method of any of the preceding claims wherein the dimensions are measured by a physician.

12. The method of any of the preceding claims wherein the first facility is a medical facility.

13. The method of any of the preceding claims wherein the second facility is a manufacturing facility.

14. The method of any of the preceding claims wherein the first facility and the second facility are the same facility.
